# EUROPÄISCHE PATENTSCHRIFT

(11) **EP 0 906 281 B1**
(45) Veröffentlichungstag und Bekanntmachung des Hinweises auf die Patenterteilung: **28.11.2001**
(21) Anmeldenummer: 97927086.5
(22) Anmeldetag: 30.05.1997
(51) Int. Cl.: C07D 211/58, C07D 221/20

(54) **VERFAHREN ZUR REINIGUNG VON STERISCH GEHINDERTEN 4-AMINO-PIPERIDINEN**
PROCESS FOR PURIFYING STERICALLY HINDERED 4-AMINO PIPERIDINES
PROCEDE DE PURIFICATION DE 4-AMINOPIPERIDINES A ENCOMBREMENT STERIQUE

(30) Priorität: 03.06.1996 DE 19622269
(43) Veröffentlichungstag der Anmeldung: 07.04.1999
(73) Patentinhaber: BASF AKTIENGESELLSCHAFT, 67056 Ludwigshafen (DE)
(72) Erfinder: JULIUS, Manfred, D-67117 Limburgerhof (DE); RUST, Harald, D-67435 Neustadt (DE); KRAUSE, Alfred, D-67346 Speyer (DE); SIEGEL, Hardo, D-67346 Speyer (DE); SIEGEL, Wolfgang, D-67117 Limburgerhof (DE); WITZEL, Tom, D-67069 Ludwigshafen (DE)
(86) Internationale Anmeldenummer: EP9702822
(87) Internationale Veröffentlichungsnummer: WO9746529

(56) Entgegenhaltungen:
- EP-A- 0 033 529
- US-A- 3 922 306
- S.E. MAFFUCCI ET AL.: "Controlling colour degradation in amines with sodium borohydride" SPECIALITY CHEMICALS, Bd. 4, Nr. 2, 1984, NEW YORK, US, Seite 38, 40, 41 XP002040922 in der Anmeldung erwähnt
- DATABASE WPI Section Ch, Week 9426 Derwent Publications Ltd., London, GB; Class A60, AN 94-216293 XP002040923 & SU 1 811 527 A (POLYMERIC MATERIALS CHEM RES INST) , 23.April 1993 in der Anmeldung erwähnt

## Beschreibung

Die Erfindung betrifft ein Verfahren zur Reinigung von rohen Piperidinen der Formel I 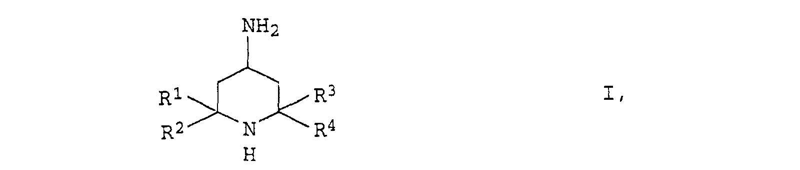 in der R¹ bis R⁴ C₁- bis C₆-Alkyl, R¹ und R² und/oder R³ und R⁴ gemeinsam eine CH₂-Kette mit 2 bis 5 C-Atomen bedeuten.

Sterisch gehinderte 4-Amino-piperidine der allgemeinen Formel I werden vielfältig eingesetzt. Sie dienen insbesondere als Zwischenprodukte bei der Herstellung von UV-Stabilisatoren für synthetische Polymere. Daher ist es von hoher Wichtigkeit, daß die Piperidine I eine möglichst geringe Eigenfärbung besitzen, die trotz geringstem Nebenproduktgehalt der Piperidine über lange Zeit beibehalten wird.

Die sterisch gehinderten 4-Amino-piperidine werden großtechnisch im allgemeinen aus Aceton oder Acetonderivaten hergestellt. Man kann I einstufig in einer katalytischen Ringschlußreaktion in Gegenwart von Ammoniak und Wasserstoff aus den Verbindungen 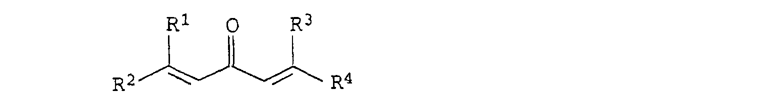 (DE 2 412 750) erhalten oder aus Triacetonaminen II 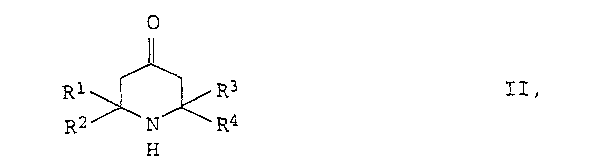 durch aminierende Hydrierung in ein oder zwei Stufen, beispielsweise katalytisch, erhalten (siehe beispielsweise DE 2 040 975, DE 2 349 962, DE 26 21 870, EP 33 529, EP 42 119, EP 303 279, EP 410 970, EP 611 137, EP 623 585 und DE 42 10 311). Die Reinigung der technisch hergestellten sterisch gehinderten 4-Aminopiperidine erfolgt im allgemeinen durch Destillation.

In den Piperidinen I, die mit üblichen Verfahren hergestellt werden, liegen jedoch noch störende Mengen an färbenden Substanzen vor oder diese werden nach kurzer Zeit gebildet.

Nun war beispielsweise aus EP 477 593 bekannt, daß man rohe N-Alkyl-di-alkanolamine farblich verbessern kann, wenn man ein Metallborhydrid zusetzt und in Gegenwart von Wasser unter bestimmten Bedingungen destilliert. Es handelt sich bei den N-Alkyl-di-alkanolaminen jedoch um eine völlig andere Substanzklasse als die sterisch gehinderten 4-Amino-piperidine der vorliegenden Erfindung. Weiterhin war aus Spec. Chem. 4(2), S. 38-41 (1984) und US 3,159,276, US 3,207,790 und US 3,222,310 bekannt, daß durch Zusatz von Natriumborhydrid zu Ethanolaminen, Ethylenaminen oder aromatischen Aminen vor oder nach einer Destillation die Farbe der Produkte verbessert werden kann. Auch in dieser Schrift werden die Piperidine I nicht erwähnt. Ein besonderes Destillationsverfahren wird nicht nahegelegt.

Im Gegensatz dazu nahm die Entwicklung von Hochreinigungsmethoden für die sterisch gehinderten 4-Amino-piperidine I einen völlig anderen Weg.

Aus DD 266 799 war bekannt, Piperidine I in Aceton-/Wasserlösung mit CO₂ umzusetzen, den Niederschlag abzutrennen, mit Aceton zu waschen, anschließend thermisch zu zersetzen und das Produkt durch Destillation zu reinigen. In SU 18 11 527 wird ein anderes Reinigungsverfahren für sterisch gehindertes 4-Amino-piperidin I beschrieben. Das verunreinigte Rohprodukt wird dabei in einem aprotischen Lösungsmittel gelöst, mit Ethylenglykol umgesetzt, das Umsetzungsprodukt destilliert und in mehreren Schritten gereinigt. Beide Verfahren sind außerordentlich aufwendig und kostenintensiv.

Aufgabe der vorliegenden Erfindung war es daher, ein kostengünstiges Verfahren bereitzustellen, mit dem hochreine farbstabile sterisch gehinderte 4-Amino-piperidine I zur Verfügung gestellt werden können, d.h. Produkte, deren geringe Eigenfärbung längere Zeit erhalten bleibt bei gleichzeitig geringem Gehalt an Nebenprodukten wie stabilisierenden Hilfsstoffen.

Demgemäß wurde das oben genannte Verfahren gefunden, das dadurch gekennzeichnet ist, daß man in einem ersten Schritt hochsiedende Substanzen und gegebenenfalls Wasser aus den rohen Piperidinen destillativ abtrennt, in einem zweiten Schritt 0,01 bis 5 Gew.-%, bezogen auf das Produkt des ersten Schrittes, eines Reduktionsmittels zusetzt und in einem dritten Schritt die Piperidine durch Destillation isoliert.

Weitere Ausprägungen der Erfindung sind den Unteransprüchen zu entnehmen.

In den sterisch gehinderten 4-Amino-piperidinen I sind die Reste R¹, R², R³ und R⁴ unabhängig voneinander bevorzugt C₁- bis C₃-Alkyl, insbesondere Ethyl oder Methyl, beispielsweise Methyl.

Als Reduktionsmittel werden im allgemeinen unter Normalbedingungen feste Substanzen, vorteilhafterweise Verbindungen MXH₄₋ₘYₘ, in denen M ein Alkalimetall, NR₄, wobei R gleiche oder verschiedene C₁-C₄-Alkylgruppen bedeuten, oder ein Äquivalent eines Erdalkalimetalles oder ein Äquivalent Zink, bevorzugt ein Alkalimetall, insbesondere Natrium oder Kalium, beispielsweise Natrium, und X Aluminium oder insbesondere Bor, Y CN oder bevorzugt H und m 1 oder insbesondere 0 darstellen, eingesetzt. Ein Beispiel ist Natriumborhydrid. In manchen Fällen haben sich auch (R^{a}O)₂TiBH₄ oder (R^{a}O)₃TiBH₄, in denen R^{a} eine C₁-C₄-Alkylgruppe bedeutet, bewährt.

Das erfindungsgemäße Verfahren kann kontinuierlich oder diskontinuierlich durchgeführt werden. Es kann bei Normaldruck oder bevorzugt bei vermindertem Druck, insbesondere zwischen 10 und 200 mbar, beispielsweise zwischen 20 und 100 mbar betrieben werden.

Die genannten Siedepunkte oder Siedebereiche beziehen sich, wenn nichts anderes angegeben ist, auf einen Druck von 40 mbar. Sie beziehen sich jeweils auf die Reinigung von 4-Amino-2,2,6,6-tetramethylpiperidin. Für andere 4-Amino-piperidine I kann der Fachmann mit einfachen Versuchen die entsprechenden Bedingungen ableiten.

Unter hochsiedenden Substanzen werden im allgemeinen solche Stoffe verstanden, die einen Siedepunkt von mindestens 35°C über dem des gewünschten Wertproduktes bei einem Druck von 40 mbar haben. Bei 4-Amino-2,2,6,6-tetramethylpiperidin werden die hochsiedenden Substanzen beispielsweise durch Destillation bei 140°C bei 40 mbar abgetrennt.

Im ersten Schritt wird das Rohprodukt destillativ von Hochsiedern und in einer besonderen Ausprägung der Erfindung von Wasser befreit. Dies geschieht im allgemeinen durch (a) Rektifikation, wobei die Hochsieder als Sumpf und das Wasser über Kopf in einem Schritt entfernt werden, oder (b) alternativ durch Abdestillieren des Rohproduktes von den Hochsiedern in geeigneten Anlagen wie Wischblattverdampfer, Fallfilmverdampfer oder Rührkessel mit aufgesetztem Kondensator und anschließendem Abdestillieren des Wassers. Das Abtrennen von Wasser ist nicht kritisch und kann beispielsweise bei 100 mbar zwischen 40 und 50°C oder entsprechend bei anderen Drucken erfolgen. Zur Abtrennung der Hochsieder wird der Destillationssumpf im allgemeinen auf eine Temperatur von bis zu 140°C (bei 40 mbar) aufgeheizt. Je weniger Hochsieder im Piperidin I vorliegen, desto bessere Ergebnisse werden erzielt.
Im allgemeinen können Hochsiedergehalte von unter 0,1 Gew.-% im Piperidin I noch toleriert werden. Besser sind jedoch Hochsiedergehalte von weniger als 0,01 Gew.-%. Der Wassergehalt ist unkritisch, üblicherweise wird er auf unter 1 Gew.-% eingestellt.

Danach wird in einem zweiten Schritt das Reduktionsmittel zugesetzt. Im allgemeinen reichen 0,01 bis 2 Gew.-% zur Erreichung eines farblosen Produktes mit den gewünschten Eigenschaften aus. Bevorzugt werden 0,01 bis 1, insbesondere 0,01 bis 0,5 Gew.-% eingesetzt. Die Zugabeart ist unkritisch. Das Reduktionsmittel wird üblicherweise in den Destillationssumpf gegeben. Das Reduktionsmittel kann in Pulverform oder als Lösung zugesetzt werden.

Nach Zugabe des Reduktionsmittels wird das Produkt destillativ gereinigt. Üblicherweise wird in bekannten Rektifikationskolonnen, wie Boden- oder Füllkörperkolonnen, bevorzugt Füllkörperkolonnen gearbeitet. Man kann mit verschiedenen Rücklaufverhältnissen arbeiten, wie der Fachmann durch kurze Vorversuche leicht herausfinden kann. Bewährt hat sich ein Verhältnis von Rücklauf zu Produktabnahmemenge von 1:1 bis 10:1, insbesondere 3:1 bis 7:1. Das Produkt 4-Amino-2,2,6,6-tetramethylpiperidin siedet bei 40 mbar bei 101°C, wie der Fachmann leicht feststellen kann (s. beispielsweise J. Polym. Sci., Part A-1, 10(11), 3295-310 (1972)); so daß es üblicherweise bei einer Temperatur von 96 bis 103°C (bei 40 mbar) gewonnen wird.

Zu dem so erhaltenen an sich farblosen Produkt kann man zusätzlich geringe Mengen an Reduktionsmittel zugeben, um die Lagerstabilität zu erhöhen. Die Reduktionsmittel sind üblicherweise die oben genannten Substanzen. Sie werden im allgemeinen in Mengen von 0,001 bis 0,02 Gew.-% zugesetzt. Üblicherweise reichen Mengen von bis zu 200 ppm Reduktionsmittel aus. In besonderen Fällen sind auch 100, 50 oder 10 ppm ausreichend.

Mit dem erfindungsgemäßen Verfahren erhält man in einfacher, kostengünstiger Weise reine sterisch gehinderte 4-Aminopiperidine I, die praktisch keine Eigenfärbung haben und bei Lagerung farbstabil bleiben bei gleichzeitig sehr geringem Gehalt an Nebenprodukten wie Stabilisierungsmitteln.

### Beispiele

Die Farbe der sterisch gehinderten 4-Amino-piperidine I wurde durch Messung der APHA-Farbzahl nach DIN-ISO 6271 bestimmt.

### Beispiel 1

Rohes 4-Amino-2,2,6,6-tetramethyl-piperidin (TAD) mit der Zusammensetzung
83,6 % Triacetondiamin (TAD)
10,0 % H₂O
1,5 % Tiefsieder
4,9 % Mittel- und Hochsieder
wurde in einem Wischblattverdampfer (200 cm² Verdampferfläche, 400 U/min) bei 40 mbar und 140°C destilliert. Bei einem Zulauf von 500 g/h (auf 100°C vorgewärmt), einer Sumpftemperatur von 106°C und einer Kopftemperatur von 89°C wurden 2560 g (92,3 %) abdestilliert. Das Destillat besaß folgende Zusammensetzung:
87,2 % Triacetondiamin (TAD)
9,9 % H₂O
1,5 % Tiefsieder
1,4 % Mittelsieder.

Die auf diese Weise von Hochsiedern befreite TAD-Rohware wurde anschließend in einer Kolonne mit 2,4 m Sulzer-CY-Packung (ca. 22 theor. Böden, Nennweite: 43 mm) bei einem Rücklaufverhältnis von 5:1 und einem Druck von 100 mbar rektifiziert. Zuerst wurden 256 g Wasser abdestilliert bei einer Kopftemperatur von 43 bis 44°C. In der Blase fügte man dem Sumpf 0,1 Gew.-% Natriumborhydrid hinzu und setzte die Rektifikation bei 40 mbar fort. Nach dem Abtrennen einer Fraktion bei einer Kopftemperatur von 89°C, die 85,9 % TAD enthielt (111 g), und einer weiteren Fraktion bei einer Kopftemperatur von 99°C, die 98,4 % TAD enthielt (240 g), wurden im Hauptlauf der Rektifikation bei einer Kopftemperatur von 99 bis 102°C 1786 g TAD mit einer Reinheit von 99,8 % (GC) erhalten. Dies entspricht einer Destillationsausbeute von 77 %.
A) Über einen Zeitraum von mindestens 5 Wochen erwies sich diese TAD-Reinware als farbstabil (APHA-Farbzahl < 50), wie die folgende Tabelle zeigt.
B) Eine Probe des nach diesem Verfahren erhaltenen TADs wurde auch unter Zusatz von 110 ppm Natriumborhydrid gelagert. Durch diesen Zusatz wurde eine für mindestens 5 Wochen praktisch ungefärbte, zusätzlich farbstabile TAD-Ware mit einer APHA-Farbzahl von < 15 erhalten.

Die Proben (A) und (B) wurden unter identischen Bedingungen bei Raumtemperatur gelagert.

| Lagerzeit in Tagen | Farbzahl (unverdünnte Ware) | |
|---|---|---|
| | (A) ohne NaBH₄-Zusatz | (B) Zusatz von 110 ppm NaBH₄ |
| 13 | 43 | 5 |
| 14 | 37 | 6 |
| 16 | 37 | 5 |
| 18 | 38 | 4 |
| 19 | 40 | 3 |
| 20 | 38 | 7 |
| 21 | 41 | 11 |
| 22 | 36 | 8 |
| 25 | 48 | 13 |
| 32 | 36 | 3 |
| 39 | 43 | 4 |

### Vergleichsbeispiel 1

2987 g Roh-TAD mit der Zusammensetzung
86,2 % Triacetondiamin (TAD)
8,7 % H₂O
ca. 1,1 % Tiefsieder
4,0 % Mittel- und Hochsieder
wurden in einer Laborapparatur in einer Kolonne mit 2,4 m Sulzer-CY-Packung (ca. 22 theor. Böden, Nennweite: 43 mm) bei einem Rücklaufverhältnis von 5:1 rektifiziert. Bei einem Druck von 100 mbar wurden zunächst 349 g als Vorlauf, der hauptsächlich Wasser enthielt, bei einer Temperatur von 43 bis 44°C abdestilliert. Anschließend wurden dem Destillationssumpf, der weniger als 0,1 % Wasser enthielt, 3,0 g (0,1 Gew.-%) Natriumborhydrid zugesetzt und die Rektifikation fortgesetzt. Nach einem Vorlauf von 222 g TAD mit einer Reinheit von 99,0 % wurden im Hauptlauf bei einem Druck von 40 mbar, einer Kopftemperatur von 97°C 2116 g TAD mit einer Reinheit von 99,7 % (GC), bei einem Wassergehalt von < 0,1 % erhalten; Destillationsausbeute: 2116 g (82 %).

Die erhaltene TAD-Reinware verfärbte sich trotz Lagerung unter Stickstoff im Dunkeln während zwei Tagen gelb (APHA-Farbzahl > 100).

### Vergleichsbeispiel 2

4208 g Roh-TAD mit der Zusammensetzung
85,6 % Triacetondiamin (TAD)
9,0 % H₂O
ca. 0,7 % Tiefsieder
4,7 % Mittel- und Hochsieder
wurden in einer Laborapparatur in einer Kolonne mit 2,4 m Sulzer-CY-Packung (ca. 22 theor. Böden, Nennweite: 43 mm) bei einem Rücklaufverhältnis von 5:1 und einem Druck von 100 bis 40 mbar rektifiziert. Nach der Abtrennung des Wassers im Vorlauf bei einer Temperatur von 43 bis 44°C und einem Druck von 100 mbar wurden im Hauptlauf bei einem Druck von 40 mbar und einer Kopftemperatur von 97 bis 103°C TAD-Reinfraktionen mit einem TAD-Gehalt von > 99,6 % erhalten (GC); Destillationsausbeute: 3022 g (84 %).

Eine Reinfraktion (TAD-Gehalt nach GC: 99,7 %) wurde zum einen ohne Natriumborhydrid-Zusatz und zum anderen nach Zusatz von verschiedenen Mengen an Natriumborhydrid wie bei Beispiel 1 gelagert und vermessen. Das Ergebnis zeigt die folgende Tabelle:

| Lagerzeit in Tagen | Farbzahl (unverdünnte Ware): (nach DIN-ISO 6271) | | |
|---|---|---|---|
| | ohne NaBH₄-Zusatz | 1000 ppm | 100 ppm |
| 1 | 238 | 207 | 225 |
| 3 | 297 | 16 | 61 |
| 4 | 289 | 12 | 35 |
| 5 | 287 | 10 | 27 |
| 6 | 286 | 9 | 32 |
| 10 | 299 | 5 | 73 |
| 11 | 297 | 4 | 72 |
| 42 | 309 | 3 | 255 |

Nachträglicher Zusatz von großen Mengen an Reduktionsmittel (0,1 % 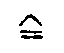 1000 ppm) erhöht zwar die Farbstabilität, jedoch auch den Hilfsstoffanteil in unerwünschter Weise.

## Patentansprüche

1. Verfahren zur Reinigung von rohen Piperidinen der Formel I 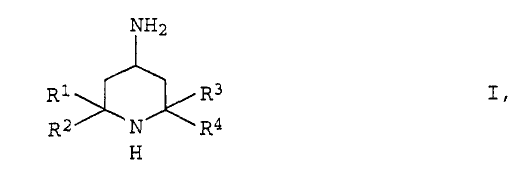 in der R¹ bis R⁴ C₁- bis C₆-Alkyl, R¹ und R² und/oder R³ und R⁴ gemeinsam eine CH₂-Kette mit 2 bis 5 C-Atomen bedeuten, **dadurch gekennzeichnet, daß** man in einem ersten Schritt hochsiedende Substanzen und gegebenenfalls Wasser aus den rohen Piperidinen destillativ abtrennt, in einem zweiten Schritt 0,01 bis 5 Gew.-%, bezogen auf das Produkt des ersten Schrittes, eines Reduktionsmittels zusetzt und in einem dritten Schritt die Piperidine durch Destillation isoliert.

2. Verfahren nach Anspruch 1, **dadurch gekennzeichnet, daß** R¹, R², R³ und R⁴ C₁- bis C₃-Alkyl bedeuten.

3. Verfahren nach Anspruch 1 oder 2, **dadurch gekennzeichnet, daß** das Reduktionsmittel eine Verbindung der Formel MXH₄₋ₘYₘ ist, in der M ein Alkalimetall, NR₄, wobei R gleiche oder verschiedene C₁-C₄-Alkylgruppen bedeuten, oder ein Äquivalent eines Erdalkalimetalles oder ein Äquivalent Zink, X Bor oder Aluminium, Y H oder CN und m 0 oder 1 bedeuten.

4. Verfahren nach einem der Ansprüche 1 bis 3, **dadurch gekennzeichnet, daß** man den durch Destillation im dritten Schritt isolierten Piperidinen I 0,001 bis 0,02 Gew.-%, bezogen auf die Piperidine, eines Reduktionsmittels zusetzt.

## Claims

1. A process for purifying crude piperidines of the formula I 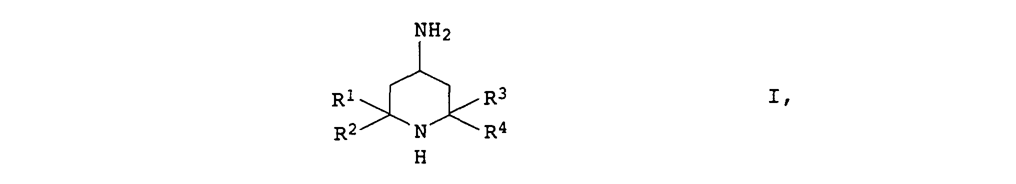 where R¹ to R⁴ are C₁-C₆-alkyl, or R¹ and R² and/or R³ and R⁴ together are a CH₂-chain of 2 to 5 carbons, which comprises, in a first step, removing high-boiling substances and, if present, water from the crude piperidines by distillation; in a second step, adding from 0.01 to 5 % by weight, based on the product of the first step, of a reducing agent; and, in a third step, isolating the piperidines by distillation.

2. A process as claimed in claim 1, wherein R¹, R², R³ and R⁴ are C₁-C₃-alkyl.

3. A process as claimed in claim 1 or 2, wherein the reducing agent is a compound of the formula MXH₄₋ₘYₘ where M is an alkali metal, NR₄, where each R is an identical or different C₁-C₄-alkyl, or one equivalent of an alkaline earth metal or one equivalent of zinc, X is boron or aluminum, Y is H or CN, and m is 0 or 1.

4. A process as claimed in any one of claims 1 to 3, wherein from 0.001 to 0.02 % by weight, based on the piperidines, of a reducing agent is added to the piperidines I isolated by distillation in the third step.

## Revendications

1. Procédé de purification de pipéridines brutes de la formule générale I 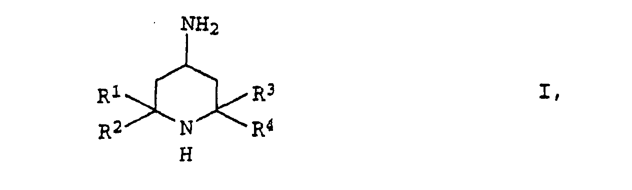 dans laquelle R¹ à R⁴ représentent des radicaux alkyle en C₁ à C₆, R¹ et R² et/ou R³ et R⁴ représentent ensemble une chaîne de CH₂ comportant de 2 à 5 atomes de carbone, **caractérisé en ce qu'**en une première étape, on sépare des pipéridines brutes, par distillation, des substances à haut point d'ébullition et éventuellement de l'eau, en une deuxième étape on ajoute un agent réducteur à raison de 0,01 à 5% en poids, par rapport du produit de la première étape et, en une troisième étape, on isole les pipéridines par distillation.

2. Procédé selon la revendication 1, **caractérisé en ce que** R¹, R², R³ et R⁴ sont des radicaux alkyle en C₁ à C₃.

3. Procédé selon la revendication 1 ou 2, **caractérisé en ce que** l'agent réducteur est un composé de formule MXH₄₋ₘYₘ, dans laquelle M est un métal alcalin, NR₄, où R sont identiques ou différents et représentent des radicaux alkyle en C₁ à C₄, ou un équivalent d'un métal alcalino-terreux ou un équivalent de zinc, X représente du bore ou de l'aluminium, Y représente H ou CN et m a une valeur de 0 ou 1.

4. Procédé selon l'une des revendications 1 à 3, **caractérisé en ce que** l'on ajoute un agent réducteur aux pipéridines I isolées par distillation dans l'étape 3, à raison de 0,001 à 0,02% en poids.
